# EUROPEAN PATENT APPLICATION

(11) **EP 3 062 250 A1**
(43) Date of publication of application: **31.08.2016**
(21) Application number: 16155977.8
(22) Date of filing: 16.02.2016
(51) Int. Cl.: G06F 19/00

(54) **SYSTEM AND METHOD FOR EFFECTIVE VISITING NURSE COMMUNICATION**

(30) Priority: 27.02.2015 US 201514633640
(71) Applicant: Honeywell International Inc., Morris Plains, NJ 07950 (US)
(72) Inventor: ANUMOLU, MallikarjunaRao, Morris Plains, NJ 07950 (US); MAHENDRA, Manoj, Morris Plains, NJ 07950 (US); RAVI, Vigneshwari, Morris Plains, NJ 07950 (US); NAIR, Manoj, Morris Plains, NJ 07950 (US); NAYAKAR, Girish, Morris Plains, NJ 07950 (US); KANNAMKATTIL, Som Appu, Morris Plains, NJ 07950 (US); PETER, Sunil, Morris Plains, NJ 07950 (US)
(74) Representative: Houghton, Mark Phillip

(57) **Abstract**

A method and apparatus that includes scheduling each of a plurality of healthcare people to visit a different at least one of a plurality of human patients in a respective residence of the patient, monitoring a vital health parameters of each of the plurality of patients, detecting a priority event within the vital health parameters of one of the plurality of patients, identifying at least one of the plurality of healthcare people that is relatively closest to the one patient and wirelessly sending the identified one healthcare person instructions to visit the residence of the one patient and a link to the vital health parameters of the one patient, along with an intimation of such to the one patient.

## Description

### FIELD

This application relates to healthcare and more particular to home healthcare.

### BACKGROUND

Systems are known to provide home healthcare workers (e.g., nurses) for visiting patients at home. Such systems typically schedule such workers based upon the perceived need of the patient.

In general, the need for such systems is based upon the age or infirmity of injured or disabled patients. Often visits to such patients are scheduled weeks in advance.

In order to improve the quality of care, monitoring equipment is often connected to in-home patients. Vital parameters may be read from a patient and automatically reported to a central server on a periodic basis.

In anticipation of a visit, a healthcare worker will often retrieve monitored parameters from the server in advance. During the visit, the worker may confirm the readings, look for additional symptoms and counsel the patient on steps to be taken before the next visit.

During or after the visit, the nurse may prepare a report regarding the visit. The report may identify the patient, the address, any findings identified during the visit and a recommendation for the next visit.

Upon returning to a central location, the report is entered through the server in the patient's file. A physician may review the file and amend or add to the report and scheduling as needed.

If the patient has additional symptoms between visits, then the patient may call the nurse and report the additional symptoms. Alternatively, the patient may visit an emergency room if the symptoms are of sufficient severity.

While such system works well, they are not very flexible and often incurs significant expense due to unnecessary emergency room visits. Accordingly, a need exists for better methods of administering home healthcare.

### BRIEF DESCRIPTION OF THE DRAWINGS

FIG. 1 illustrates a block diagram of a system in accordance herewith;
FIG. 2 is a flow chart of steps performed by a processor of FIG. 1 for providing data to a healthcare worker;
FIG. 3 is a flow chart of steps performed by a processor of FIG. 1 for prioritizing visits by a healthcare worker; and
FIG. 4 is a flow chart of steps performed by a processor of FIG. 1 for expediting visits by a healthcare worker.

### DETAILED DESCRIPTION

While disclosed embodiments can take many different forms, specific embodiments thereof are shown in the drawings and will be described herein in detail with the understanding that the present disclosure is to be considered as an exemplification of the principles thereof as well as the best mode of practicing same, and is not intended to limit the application or claims to the specific embodiment illustrated.

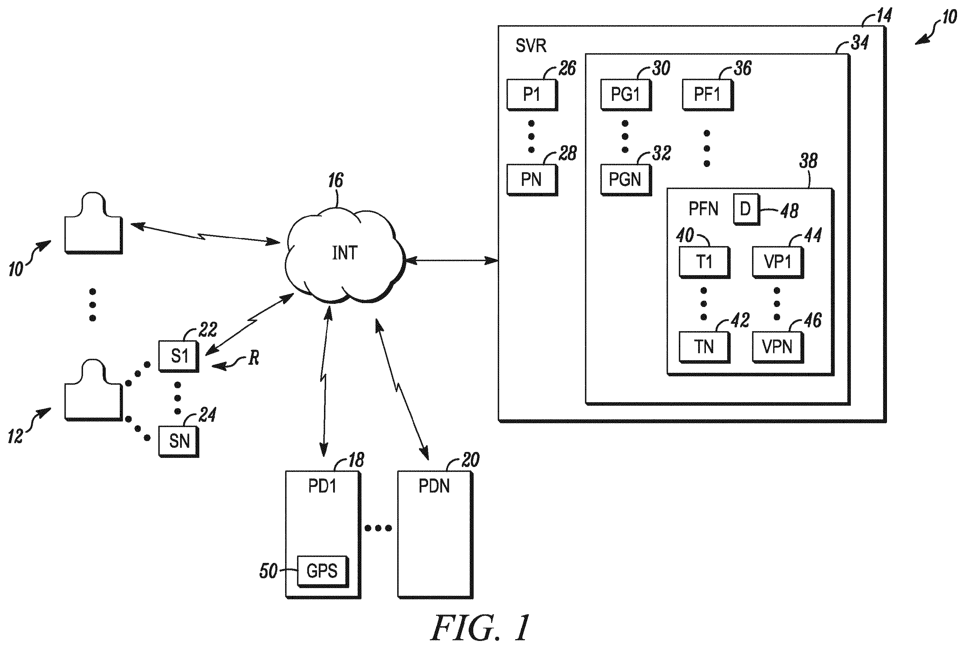

FIG. 1 is a block diagram of a healthcare system 10 shown generally in accordance with an illustrated embodiment. Included within the system are a number of human patients 10, 12 coupled to a healthcare server 14 through the Internet 16. Each of the patients may be located in a respective residence R of the patient.

Also included within the system is a number of portable wireless devices 18, 20 (e.g., smartphones) carried by a respective human healthcare worker. The portable devices may be coupled to the healthcare server via the Internet.

The vital signs (i.e., parameters) of each of the patients (e.g., heart rate, blood pressure, blood oxygen level, etc.) are monitored by one or more sensors 22, 24. The sensors are, in least in part, wirelessly coupled to the healthcare server through the Internet.

Included within the healthcare server, each of the sensors and portable devices may be one or more processor apparatus (processors) 26, 28, each operating under control of one or more computer programs 30, 32 loaded from a non-transient computer readable medium (memory) 34. As used here, reference to a step performed by a computer program is also reference to the processor that executed that step.

Under the illustrated embodiment, a measurement processor within each of the sensors may read a sensing element within the sensor and report the reading to a corresponding measurement processor within the healthcare server. The processor within the server may save the reading into a corresponding file 36, 38 maintained for each of the patients.

The patient file may include a number of fields unique to the patient. For example, one of the fields 48 may include one or more diagnosis (conditions) of the patient. Additional fields may include identifiers of the vital parameters that need to be monitored that are relevant to that condition and along a set of most recently measured vital parameters 44, 46 of the patient. A respective set of threshold values 40, 42 may be saved in the file for each monitored parameter.

Diagnosis of patient condition(s) may be provided by a physician. Based upon the diagnosis, each of the patients may be assigned to a respective healthcare worker associated with one of the portable devices. The assignment may include a frequency of visit to the residence of the patient and a set of vital parameters that are to be monitored as part of the treatment of the patient. The assignment of a healthcare person for each visit may be based upon availability or upon a treatment specialty of the healthcare person.

A scheduling processor may establish an initial visitation schedule for each patient and send a notification to the assigned healthcare workers. As part of that process, the scheduling processor may select a first patient according to some criteria (e.g., alphabetically, locale, etc.) and then a first healthcare worker based upon another criteria (e.g., specialty, availability, etc.). The process may be repeated round-robin until each patient is assigned to at least one healthcare worker. The visit may be scheduled on a repeating basis (e.g., biweekly, weekly, monthly, etc.) and for a particular day of a week, month and/or time of day.

The initial schedule may be revised based upon the locales of the patients. For example, patients in one particular geographic area may be preferentially assigned to a healthcare worker in that area to minimize travel distances.

The established schedule may be forwarded to each healthcare worker. The schedule may be accompanied with a patient summary including patient diagnosis, monitored parameters and most recent vital signs.

The healthcare worker may visit the patient in accordance with the schedule. During the visit, the healthcare worker may confirm vital signs. During the visit, the healthcare worker may also interview the patient to determine the general well-being of the patient and to confirm existing symptoms and identify any new symptoms. Upon completion of the interview, the healthcare worker may prepare a report and forward the report for inclusion in the patient file.

Under one illustrated embodiment, an alerting processor of the healthcare server operates in the background to detect critical events with respect to the vital signs of each of the patients and to route the closest healthcare worker to the residence of the patient. In this regard, the alerting processor may sequentially identify and retrieve the latest vital signs of each patient (e.g., heartbeat, blood pressure respiration rate, etc.) and compare them with the threshold values assigned to the patient. If one or more of the vital signs of a particular patient exceeds a corresponding threshold value, then the alerting processor determines that an urgent need exists and expedites a visit to the patient by one of the healthcare workers.

Based upon the determination of an urgent need, a location processor may identify one of the healthcare workers who is closest the residence R of the identified patient with the urgent need. As a first step, the location processor may request a geographic location from each of the portable devices. In this regard, each of the portable devices may have a global positioning system (GPS) device 50 that determines the real time location of each healthcare worker. The GPS device of each portable device may periodically report the location of the healthcare worker to the server or respond to a real time location request from the location processor. If the portable device periodically reports its location, then the latest location may be saved within a respective location file for each healthcare worker within memory of the healthcare server.

In either case, the location processor determines the geographic location of each healthcare worker and compares the location with the geographic location of the patient in urgent need. The healthcare worker having the smallest relative distance between the healthcare worker and the patient in urgent need may be selected to visit the patient.

In addition to selecting a healthcare worker to visit the patient, a records processor may retrieve and send the contents of the patient file including the latest vital parameters of the patient to the portable device of the selected healthcare worker. The patient file and latest vital parameters may appear as an attachment to the urgent visit request sent to the selected healthcare worker or as a link embedded into the request.

The location processor may also send a map to the healthcare worker showing the current location and location of the patient. As above, the map may be provided as an attachment or as a link attached to the request.

The system described above with respect to FIG. 1 differs from prior art systems in a number of regards. For example, currently used practices require traveling healthcare workers to contact a human tele-nurse to identify who they need to see and to obtain the vitals data for that patient. Visiting healthcare workers need to figure out the exact location and address of each visited patient. Patients whose vital signs are more deviated from normal are not differentiated from other patients whose vital signs are less deviated, so both kinds of patients are given the same priority in providing care. Currently, there is no notification mechanism for notifying a nurse that a nearby patient is in distress.

In contrast, the system described above with respect to FIG. 1 solves these problems. For example, visiting nurses (e.g., healthcare workers) are given access to mobile apps that have Internet access to patient's health data (otherwise known as LifeStream data) which provides a way for the worker to quickly see the vital information of a current patient.

The automation of the scheduling of the home visits is adjusted dynamically based upon a comparison of currently available vital signs with respective threshold values. In this regard, each vital sign (e.g., heart rate, respiration, blood pressure, blood oxygen level, etc.) may be given a number of respective threshold values. The highest level thresholds are for life threatening conditions and are given the highest (1^{st} Priority). A second level of threshold values (2^{nd} Priority) are those which suggest a serious deviation from normal and represent a serious potential health problem. A third and fourth levels (Normal Priority and Lesser Priority) are associated with normal deviations and are normally addressed through the normal scheduling process.

However, upon detecting a deteriorating vital sign, the alert processor automatically sends notification to one of the healthcare workers. This notification may be sent to whoever is nearby to the patient's location.

The system of FIG. 1 consists of a portable or otherwise mobile device having an app which has access to LifeStream vital sign data. The data is provided to the mobile device under the urgent priority discussed above and under the lesser priorities.

Using the LifeStream vital sign data, the automated telehealth nurse (scheduling processor and alerting processor) can assign patients to healthcare workers or assign healthcare workers to patients in urgent need based upon the real time needs of the patient. With the help of the mobile apps, a visiting nurse or other healthcare worker is able to know whom they need to visit and also that they have the most current and past vital sign data of that particular patient.

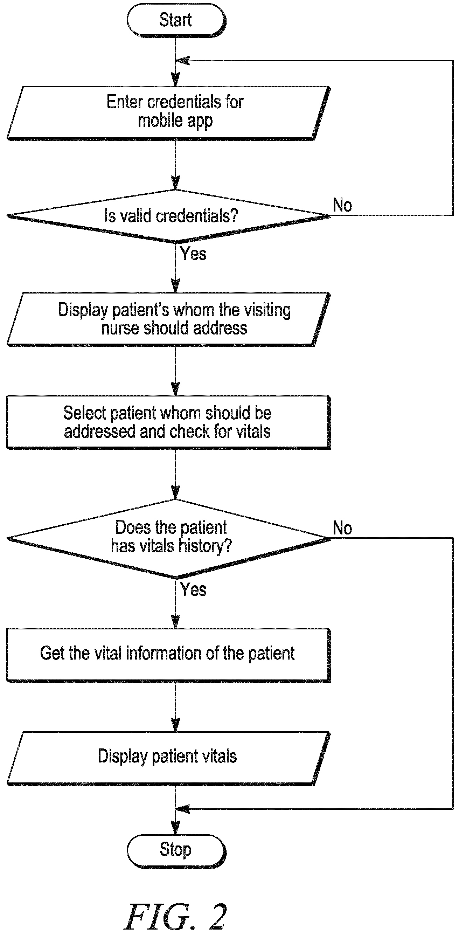

FIG. 2 depicts a flow chart of steps performed by a patient data delivery processor based upon an assignment. Once a patient has been assigned, the visiting nurse or healthcare worker obtains access to the mobile app through the portable device which can fetch the patient's data based upon the credentials of the worker. Accordingly, the process is simple for the worker to use, to know who to visit and to be able to review the vital information via the data display app instead of having to contact a human tele-health nurse and request the sending of the data.

As shown in FIG. 2, the healthcare worker enters his/her credentials via the reporting app executing on the portable device. A corresponding app within the healthcare server verifies the credentials and displays a list of patients that the worker should address via a visit that day. The worker may select patients one-by-one that are to be seen. The app inquires if the patient has a vital sign history in a patient file, gets the vital sign information and displays the vital sign parameters of each patient on a screen of the portable device.

Occasionally, the vital signs of a patient may unexpectedly deviate from normal ranges. In the case where a patient's vital signs have deteriorated, a notification is received by the visiting nurse or healthcare worker who is close to the location of the patient along with a route map, so that they can attend to the patient quickly and care can be given. The alerts based upon the vital signs can be segregated into sub categories including high, moderate and low priorities. Due to these categorizations, visiting nurses are able to attend patients who are in need first. During or after visiting the patient (with the help of the mobile device app), the visiting nurse is able to see the entire vital history of that patient which helps her/him to analyze the patient problems and communicate to the clinician for further assistance which can provide better results.

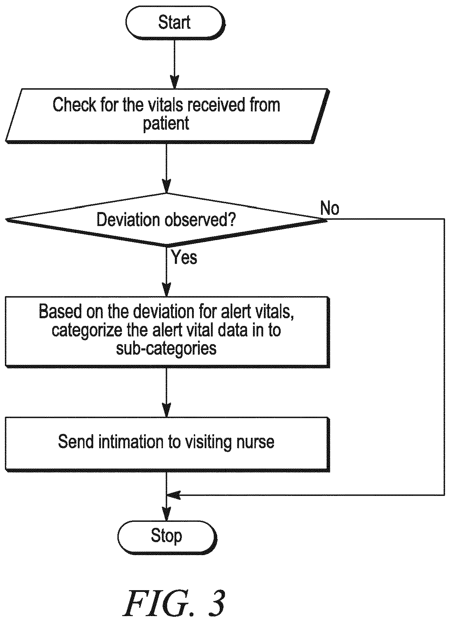

FIG. 3 depicts the overall operation of categorization by a categorization processor. Under this process, the vital signs of each patient are classified based upon the one or more thresholds provided for each respective vital sign. This classification helps to provide care quickly to patients who are really in need. Visiting workers can visit some patients on an early schedule instead of a normal schedule in the case of patients who have a greater priority and can take appropriate actions such as retaking vitals, providing proper precautions and/or providing education to patients directed to avoiding depression. This process functions to provide quick attention to those patients who really need care.

As shown in FIG. 3, the categorization processor retrieves and reviews the vital signs received from each patient. The categorization processor may determine a norm based upon average values and identify deviations from this norm. Based upon the deviations, the categorization processor may categorize the visit. If the categorization is of an intermediate level, then the visit may be rescheduled with the same healthcare worker originally scheduled to visit the patient. In this case, the visit is simply scheduled via the scheduling processor for an earlier time or date. Rescheduling in this case causes the scheduling processor to send a notice or intimation to the healthcare worker requesting the earlier visit.

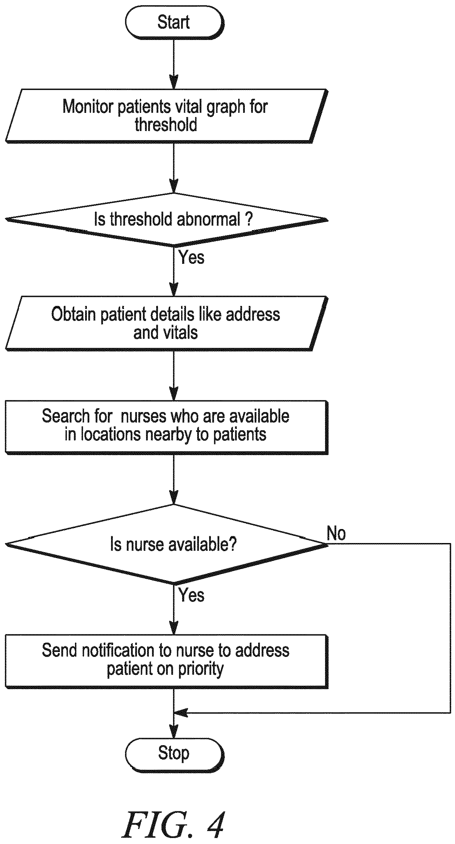

On the other hand, the vital signs and parameters may also deviate to levels that represent a dangerous level for the patient. When the trend of the patient's vital signs extend too far up or down from the norm for the patient, then the values may exceed one or more of the first and second priority levels and an intimation/notification is sent to a healthcare worker by first searching for a healthcare worker who is nearest the patient, so that care may be provided on a greatly expedited basis. This process is depicted in FIG. 4.

As shown in FIG. 4, the alert processor may monitor each patient's vital signs for deviations that approach one or more threshold values. If the current vital signs exceed the intermediate or lower threshold thereby implicating one or both of the first or second priorities, then the processor may obtain patient details such as the address and associated vital signs and search for healthcare workers who are available in locations near the patient. If a healthcare worker is identified, then notification is sent to the identified worker to address the patient's health on a priority basis.

In general, the system includes apparatus that performs the steps of scheduling each of a plurality of healthcare people to visit a different at least one of a plurality of human patients in a respective residence of the patient, monitoring a vital health parameters of each of the plurality of patients, detecting a priority event within the vital health parameters of one of the plurality of patients, identifying at least one of the plurality of healthcare people that is relatively closest to the one patient and wirelessly sending the identified one healthcare person instructions to visit the residence of the one patient and a link to the vital health parameters of the one patient, along with an intimation of such to the one patient.

Alternatively, the system includes a healthcare server that schedules each of a plurality of healthcare people to visit a different at least one of a plurality of human patients in a respective residence of the patient, a processor of the healthcare server that monitors vital health parameters of each of the plurality of patients, a processor or the healthcare server that detects a priority event within the vital health parameters of one of the plurality of patients, a processor or the healthcare server that identifies at least one of the plurality of healthcare people that is relatively closest to the one patient and a processor or the healthcare server that wirelessly sends a notification to the identified one healthcare person including instructions to visit the residence of the one patient and a link to the vital health parameters of the one patient, along with an intimation of such to the one patient.

Alternatively, the system includes a healthcare server, a database of the healthcare server that contains healthcare records of each of a plurality of human patients, a processor of the healthcare server that schedules each of a plurality of healthcare people to visit a different at least one of a plurality of human patients in a respective residence of the patient based upon the healthcare records, a processor of the healthcare server that monitors current vital health parameters received from each of the plurality of patients, a processor or the healthcare server that detects a priority event within the vital health parameters of one of the plurality of patients, a processor or the healthcare server that identifies at least one of the plurality of healthcare people that is relatively closest to the one patient and a processor or the healthcare server that wirelessly sends a notification to a portable device carried by the identified one healthcare person including instructions to visit the residence of the one patient and a link to the vital health parameters of the one patient, along with an intimation of such to the one patient.

From the foregoing, it will be observed that numerous variations and modifications may be effected without departing from the spirit and scope hereof. It is to be understood that no limitation with respect to the specific apparatus illustrated herein is intended or should be inferred. It is, of course, intended to cover by the appended claims all such modifications as fall within the scope of the claims. Further, logic flows depicted in the figures do not require the particular order shown, or sequential order, to achieve desirable results. Other steps may be provided, or steps may be eliminated, from the described flows, and other components may be add to, or removed from the described embodiments.

## Claims

1. A method comprising:
scheduling each of a plurality of healthcare people to visit a different at least one of a plurality of human patients in a respective residence of the patient;
monitoring a vital health parameters of each of the plurality of patients;
detecting a priority event within the vital health parameters of one of the plurality of patients;
identifying at least one of the plurality of healthcare people that is relatively closest to the one patient; and
wirelessly sending the identified one healthcare person instructions to visit the residence of the one patient and a link to the vital health parameters of the one patient, along with an intimation of such to the one patient.

2. The method as in claim 1 further comprising displaying the instructions on a portable wireless device of the healthcare person.

3. The method as in claim 2 further comprising displaying a map of a location of the one patient.

4. The method as in claim 3 further comprising displaying a condition of the at least one patient on a display of the portable device carried by the at least one healthcare person.

5. The method as in claim 1 wherein the step of detecting a priority event further comprises comparing one or more of the vital parameters with a respective threshold value.

6. The method as in claim 1 further comprising a global positioning system within each of the plurality of portable devices determining a position of the portable device.

7. The method as in claim 6 further comprising a processor of each of the plurality of portable devices periodically reporting its position to a healthcare server.

8. The method as in claim 1 further comprising attaching at least one health parameter sensor to each of the plurality of human patients.

9. The method as in claim 8 further comprising a processor within each of the plurality of sensors reporting a measured vital health parameter to a healthcare server.

10. An apparatus comprising:
a healthcare server that schedules each of a plurality of healthcare people to visit a different at least one of a plurality of human patients in a respective residence of the patient;
a processor of the healthcare server that monitors vital health parameters of each of the plurality of patients;
a processor or the healthcare server that detects a priority event within the vital health parameters of one of the plurality of patients;
a processor or the healthcare server that identifies at least one of the plurality of healthcare people that is relatively closest to the one patient; and
a processor or the healthcare server that wirelessly sends a notification to the identified one healthcare person including instructions to visit the residence of the one patient and a link to the vital health parameters of the one patient, along with an intimation of such to the one patient.

11. The apparatus as in claim 10 further comprising a display of the portable wireless device that displays the notification.

12. The apparatus as in claim 11 further comprising a map of a location of the one patient displayed on a screen of the portable device of the at least one healthcare person.

13. The apparatus as in claim 11 further comprising a processor that display a condition of the one healthcare person on the screen of the portable device.

14. The apparatus as in claim 10 wherein the processor that detects a priority event further comprises a processor that compares one or more of the vital parameters with a respective threshold value.

15. The apparatus as in claim 10 further comprising a global positioning system within each of the plurality of portable devices that determines a position of the portable device.
